# EUROPEAN PATENT SPECIFICATION

(11) **EP 3 606 476 B1**
(45) Date of publication and mention of the grant of the patent: **11.08.2021**
(21) Application number: 18720976.2
(22) Date of filing: 06.04.2018
(51) Int. Cl.: A61F 2/40, A61F 2/30

(54) **TOTAL SHOULDER PROSTHESIS**
KOMPLETTSCHULTERPROTHESE
PROTHÈSE D'ÉPAULE TOTALE

(30) Priority: 07.04.2017 EP 17165554
(43) Date of publication of application: 12.02.2020
(73) Proprietor: Kyon AG, 8005 Zürich (CH)
(72) Inventor: TEPIC, Slobodan, 8057 Zürich (CH)
(74) Representative: Weiss, Wolfgang
(86) International application number: PCT/EP2018/058847
(87) International publication number: WO 2018/185278

(56) References cited:
- EP-A1- 0 884 032
- WO-A1-2007/041879
- WO-A1-2013/030668
- FR-A1- 2 545 352
- FR-A5- 2 103 145
- GB-A- 2 405 346
- US-A- 3 815 157

## Description

### Field of the invention

The invention relates to a total prosthesis for the shoulder joint. It is of the so-called reverse type with a concave component in the humerus and a convex component on the scapula.

### Background

Total shoulder arthroplasty is currently the fastest growing joint replacement. This in part is due to improved clinical performance of the implants, but also due to increasing acceptance of the reverse type of the prosthesis. In the anatomic type prosthesis, the humeral head is replaced by a prosthetic head of similar size and shape, most commonly sitting on the proximal end of a humeral stem. The glenoid cavity is also replaced by an implant of very similar shape to the natural one, commonly fixed by screws and adapted for the secondary bone ingrowth.

In the reverse type prosthesis, the shape and extent of the articulation is very similar, but simply reversed: the proximal humerus now has a shallow cavity and the scapula carries a rather flat head of large radius. This shifts the center of rotation into the scapula - it is not clear if this is of help for the clinical outcomes or not, but the range of movement is generally increased.

There are still issues with the current procedures: luxations, wear of the articulation and failure of permanent anchorage to the bone.

GB 2 405 346 A, FR 2 103 145 A5, FR 2545 352 A1 and US 3 815 157 A each disclose a reverse shoulder prosthesis comprising a concave component adapted for attachment to the proximal humerus, and a convex component comprising a spherical head and a bone anchor adapted for attachment to the scapula, wherein the concave component comprises a recess adapted to receive the spherical head and providing a coverage of more than 180° of the spherical head.

The snap fit connection of the concave and the convex components during surgery can be difficult. Patent document FR 2 103 145 A5 discloses to make the concave component of two parts to render the snap fit connection easier.

The present invention, with its radical departure from conventional approaches, addresses most of the problems of this important orthopedic intervention. Additionally, it can shorten the surgery time and make the procedure significantly less invasive.

### Description of the invention

The invention discloses a total shoulder prosthesis of the reverse type. The concave component, fixed in the proximal humerus below the resection plane of the humeral head, is a deep cup, covering about 180° to about 200°, of the prosthetic head fixed to the scapula. It is very similar to the acetabular cups of the total hip prosthesis and preferably fixed by press fit into reamed-out cancellous bony bed, followed by secondary stabilization by bone ingrowth.

The convex component is a spherical head very similar to that of the femoral replacement in the total hip arthroplasty. It is attached to a scapula anchor, which may be shaped as a tubular screw and inserted into the scapula through the subchondral bone of the glenoid cavity and then along the long axis of the scapula, partially cutting into all three flat bones forming the triad of the scapula. The prosthesis of this invention offers great range of impingement-free movement, superior stability and straightforward, time-saving surgery. It is suitable for use in human and veterinary medicine.

Thus, one aspect of the present invention relates to a total shoulder prosthesis of the reverse type comprising:
(i) a concave component 300 adapted for attachment to the proximal humerus , and
(ii) a convex component comprising a spherical head 200 and a bone anchor 100 adapted for attachment to the scapula,
wherein the concave component 300 comprises a recess 301 adapted to receive the spherical head 200 and wherein the concave component 300 provides a coverage of about 200° of the spherical head 100.

A method for shoulder surgery comprises implanting a prosthesis as described above into a subject in need thereof.

The total shoulder prosthesis of this invention places a deep, spherical articulation inside the humeral bone, with its center just proximally to its natural location even though it is of reverse type. Conventional reverse-type shoulder prosthesis place the center of rotation very proximally, with a large radius flat head affixed to the scapula. Stability of either anatomical or reverse-type prosthesis is limited by their shallow articulations. The prosthesis of this invention provides superior stability and, by its refined construction relying on the best materials and the geometry of the articulation, extremely low wear.

Anchorage of both components is unique for this joint, is surgically simplified and secure. A significant time saving in surgery is expected with lower costs of both the implants and surgical fees.

In the following, preferred embodiments of the invention are described in reference to the accompanying Figures.
Figure 1 shows a perspective and planar view of the scapular components of a preferred embodiment of the prosthesis according to the present invention.
Figure 2 shows perspective views of the humeral cup of a preferred embodiment of the prosthesis according to the present invention.
Figures 3 to 11 show the surgical steps of implanting the prosthesis according to the present invention.
Figures 12 and 13 show an embodiment of a humeral cup implanted into a dog humerus.

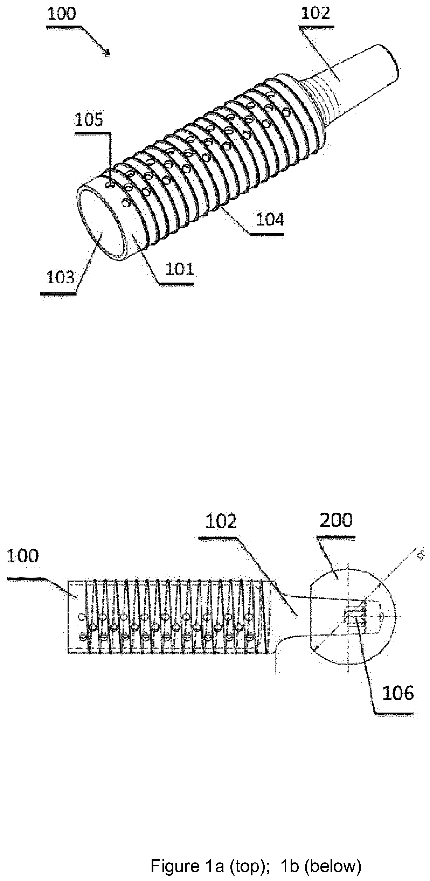

Figure 1a shows a perspective view of the scapular anchor screw 100. The cylindrical, tubular body 101 of the anchor screw carries the conical neck 102 on its distal end. Hollowed out core 103 leaves a large proportion of the bone in situ. Between the threads 104 there are numerous holes 105 facilitating rapid integration into bone and revascularization of the bone in the core of the anchor screw. Only 3 columns of the these holes are shown on this drawing, but preferably they run around the full circumference of the screw. Preferred material for the anchor screw 100 is one of titanium alloys, most preferably titanium-aluminium-niobium, which has high strength, but also exceptional biocompatibility. For improved bone integration it may be coated by porous titanium and/or hydroxy-apatite.

Figure 1b shows an orthogonal view of the anchor screw 100 and the scapular head 200 fitted to the conical neck 102. At the end of the conical neck 102, the anchor screw is provided with a screwdriver recess 106. The anchor screw neck section may be cannulated to allow its insertion over a guide pin using a cannulated screwdriver. The scapular head may be made from one of suitable metal alloys, e.g. cobalt-chromium, or more preferably from a high strength ceramic, e.g. alumina, zirconia or their blend.

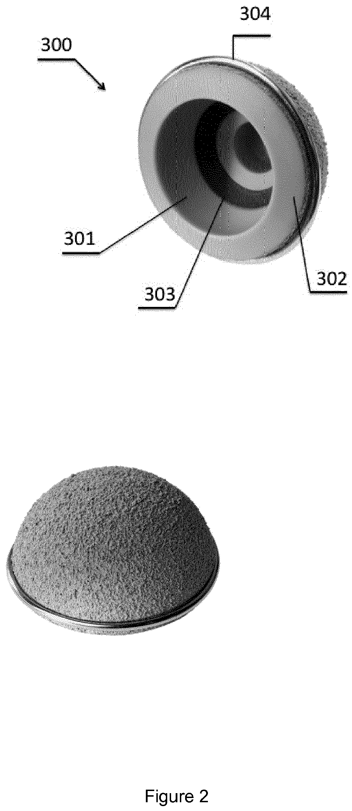

Figure 2 shows perspective views of the humeral cup 300. The recess 301 is adapted to receive the spherical head 200. It covers the head of about 180 to about 200° for greater resistance to luxation, but with still adequate range of impingement-free movement. The body 302 of the cup can be made from a biocompatible polymer such as polyether ether ketone (PEEK) and at the annular contact to the head may be provided with a ring 303, made from a reinforced material, particularly a reinforced PEEK ring, e.g. a carbon fiber reinforced PEEK ring 303. Wear of this ring when articulated with a ceramic head is extremely low. The back side of the cup, i.e. the side distal to the convex component, may be coated with a porous layer of titanium and/or hydroxy-apatite. For radiographic identification, the cup may be provided with a radiographic tag, e.g. a metal ring 304 at its equator.

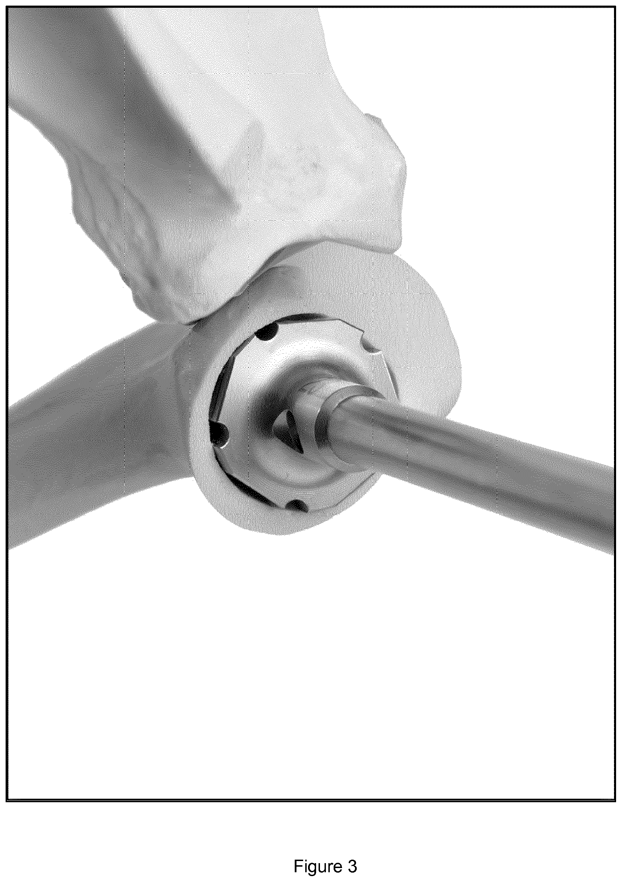

Figures 3 to 11 show a sequence of surgical steps for implantation of the shoulder prosthesis according to this invention. This sequence was made on a plastic model of a dog shoulder joint, which has a very similar anatomy to the human shoulder. The invention is equally well applicable to humans and certain animal species, dogs in particular.

Figure 3 shows reaming of the hemispherical cavity in the humerus. The head of the humerus was removed by a planar osteotomy just above the transition from the head into the neck.

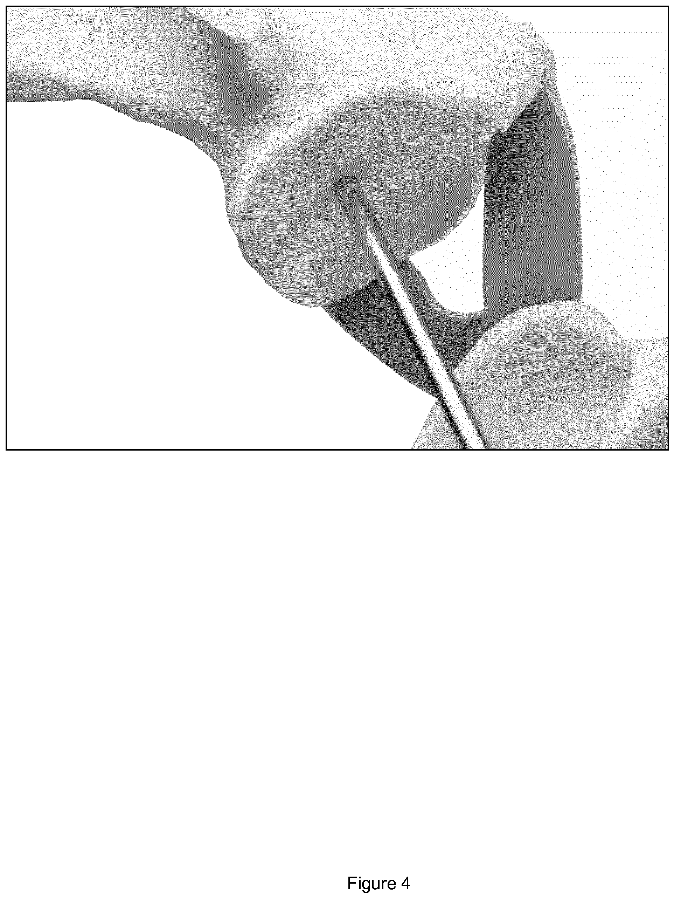

Figure 4 shows insertion of guide pin into the scapula. The starting point is at the center of the glenoid cavity; the orientation is along the line where the three planar wings of the scapula meet. Finding the proper orientation can be aided by a minor surgical exposure of the bone behind the glenoid cavity just over its edge. Aiming at approximately 90 degrees to the glenoid cavity is an alternative.

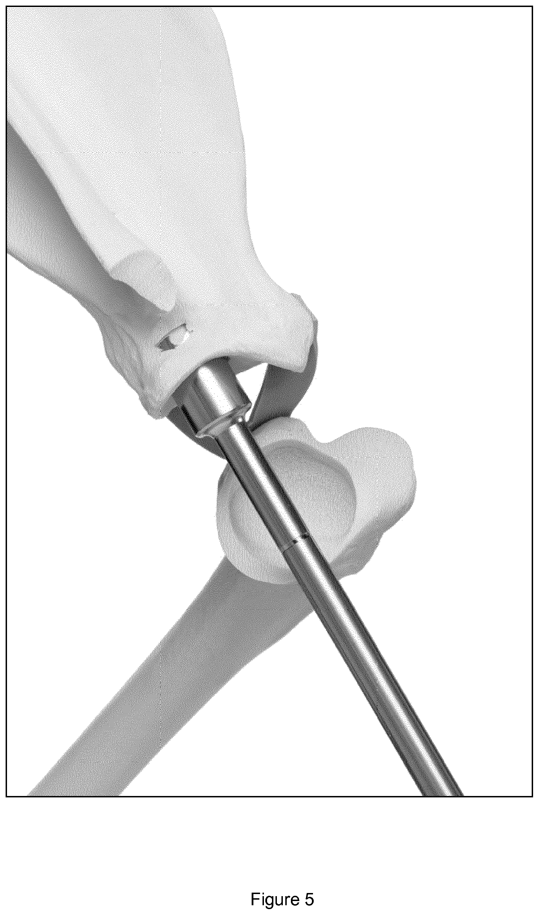

Figure 5 shows the first step of coring the cylindrical cut into the scapula with a short cannulated corer.

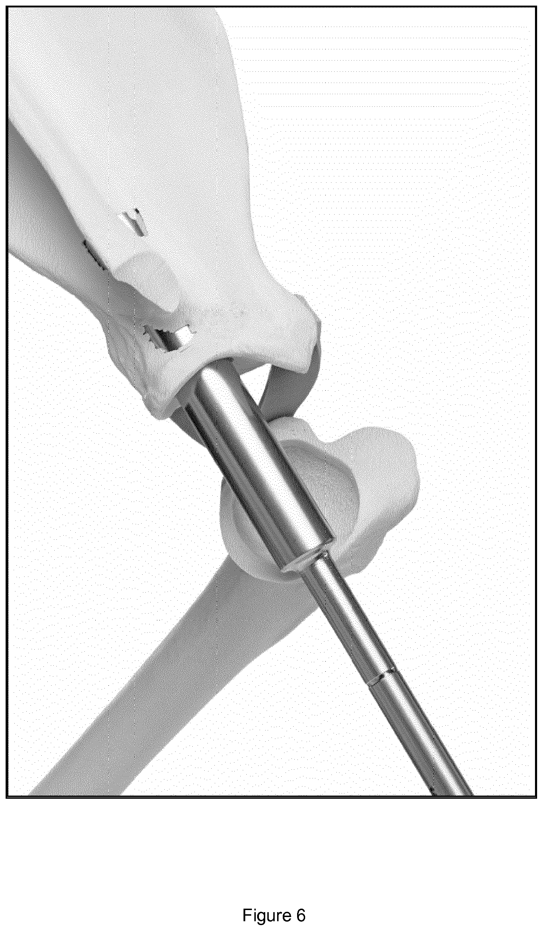

Figure 6 shows the second step of cutting deeper into the scapula with a longer cannulated corer. The length of this cutter prevents precise guidance over the guide pin, but once the first, short cutter was used to precisely center the cut over the guide pin, this long instrument can be safely used to finish the coring.

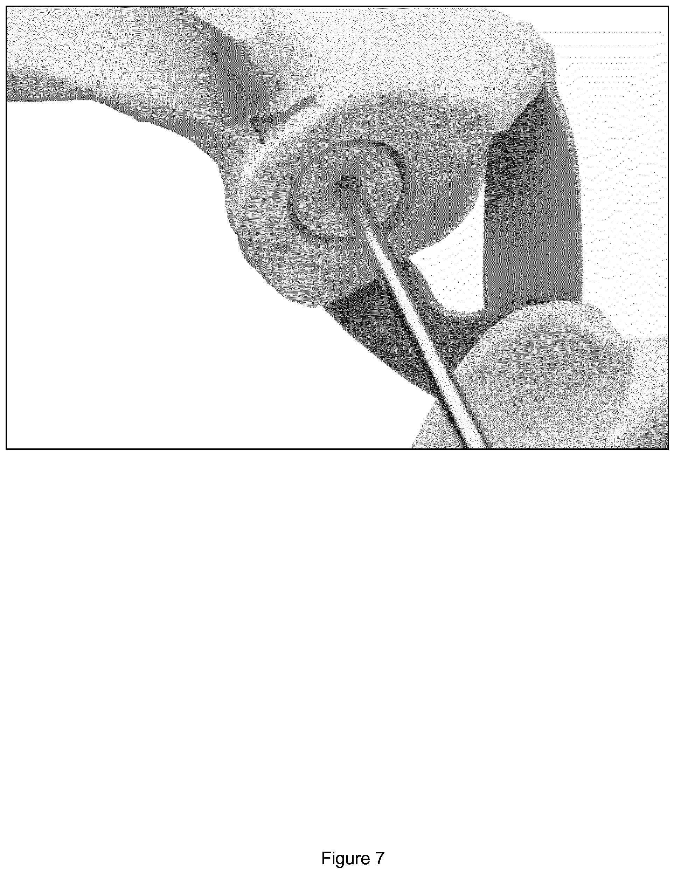

Figure 7 shows the view into the glenoid cavity after the coring for the implant was finished. Note the cut exiting the bone behind the glenoid subchondral bone, before it enters into the spine of the scapula.

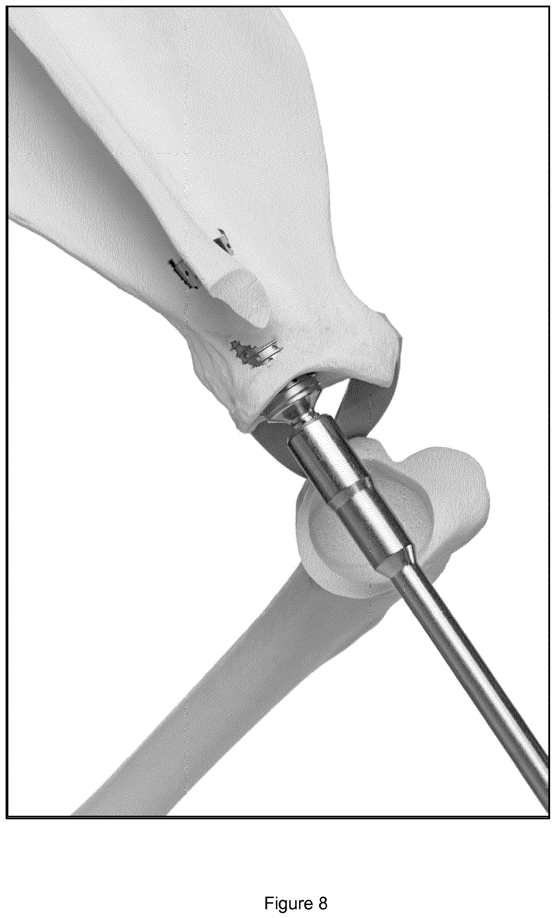

Figure 8 shows insertion of the scapular anchor screw. The screw is seated in the scapula by entering and exiting from the bone at multiple locations - periosteal reaction of the bone along those cuts is expected to lead to a rapid and strong integration of the implant.

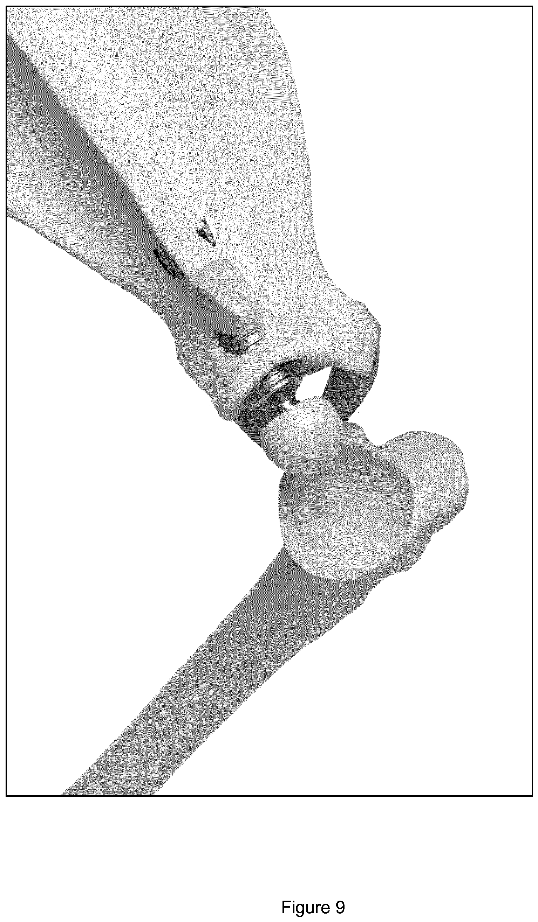

Once the screw is inserted the head of the prosthesis is attached to the anchor, figure 9, via precise conical fit. Length of the neck can be changed by either depth of anchor insertion or by choice of the heads with different recess depths, as is routinely done for e.g. total hip replacement.

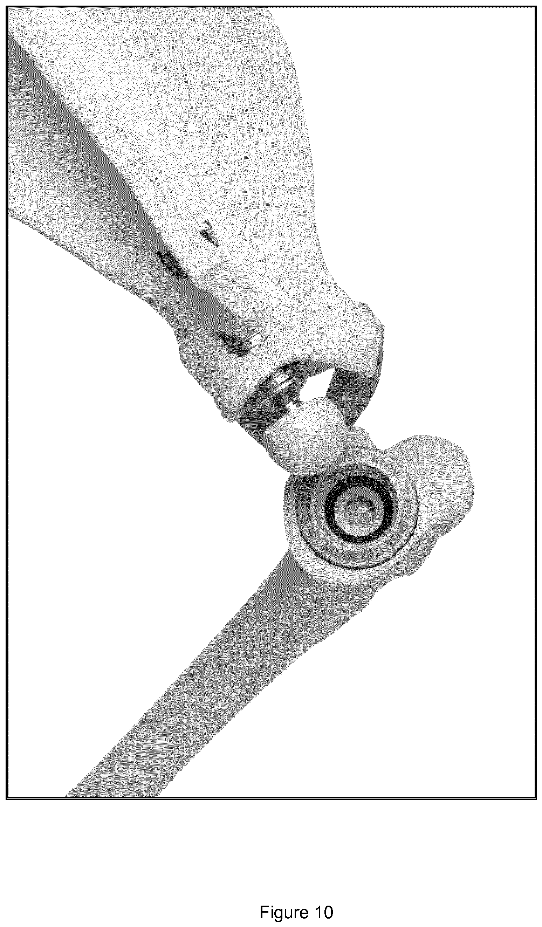

Figure 10 shows the humeral cup inserted into its prepared cavity. This is preferably a cementless type, but an alternative is fixation of the cup by bone cement.

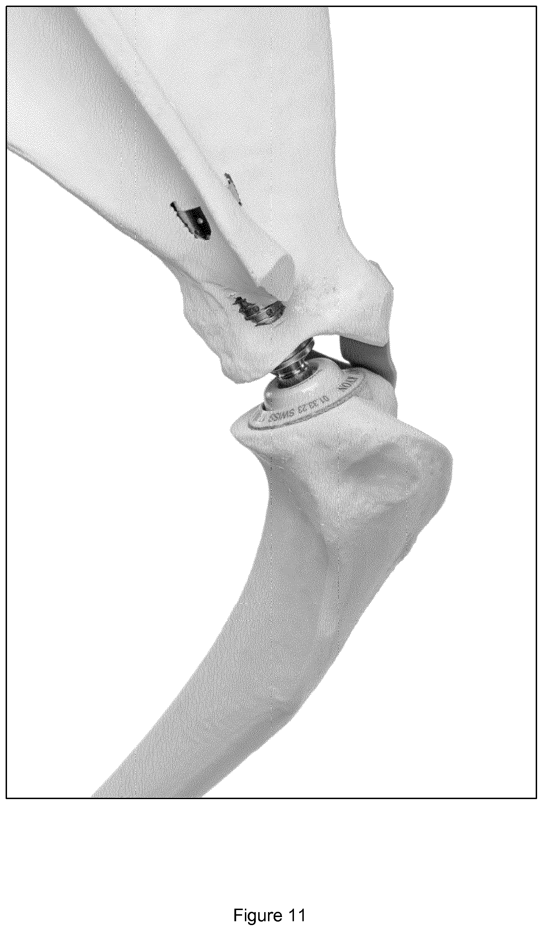

Figure 11 shows the reduced total shoulder prosthesis. Because the neck of the scapular component is of a small diameter compared to the head diameter, the prosthesis has a very large range of impingement-free movement. There is little bending expected on the anchor - indication for that is the very shallow glenoid cavity, which could not resist large variations in joint force vector.

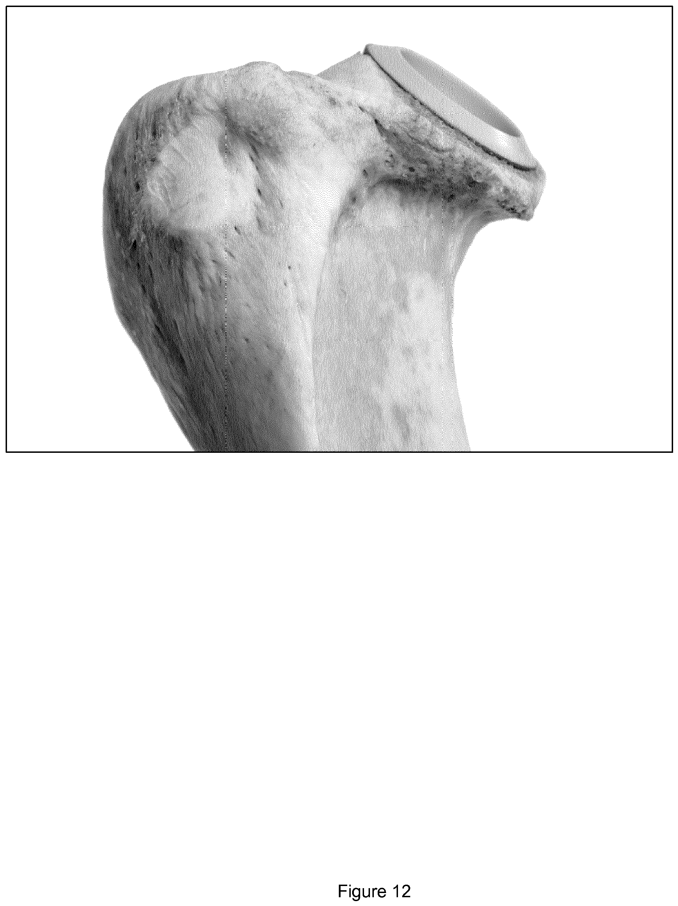

Figure 12 shows a tangential view of the humeral cup inserted in a dog cadaver. Quality of cancellous bone is very good in this region and a stable fixation without a conventional stem should be reliably achieved.

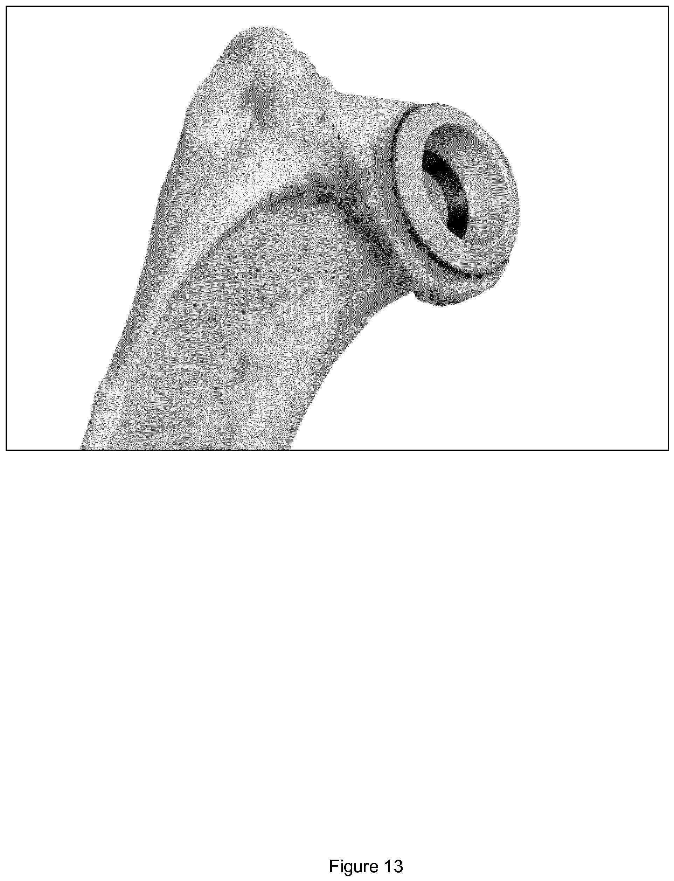

Figure 13 is perspective view of the humeral cup demonstrating its appropriate size and placement.

## Claims

1. A total shoulder prosthesis of the reverse type comprising:
(i) a concave component (300) adapted for attachment to the proximal humerus , and
(ii) a convex component comprising a spherical head (200) and a bone anchor (100) adapted for attachment to the scapula,
wherein the concave component (300) comprises a recess (301) adapted to receive the spherical head (200) and **characterized in that** the concave component (300) provides a coverage of about 180° to about 200° of the spherical head (200).

2. The prosthesis of claim 1,
wherein the concave component provides a coverage of about 180° to about 190°of the spherical head (200).

3. The prosthesis of claim 1 or 2,
wherein the concave component (300) has a body (302) made from a biocompatible polymer such as polyether ether ketone (PEEK).

4. The prosthesis of any one of claims 1-3,
wherein the concave component (300) is provided with a ring (303) made from a reinforced material in the zone of contact with the spherical head 200,
wherein the reinforced material is e.g. a reinforced PEEK.

5. The prosthesis of any one of claims 1-4,
wherein the back side of the concave component (300) is at least partially coated with a porous layer, in particular a porous layer of metal, e.g. titanium, and/or ceramic material, e.g. hydroxy-apatite.

6. The prosthesis of any one of claims 1-5,
wherein the spherical head (200) is made from metal, e.g. from a metal alloy such as cobalt-chromium, or from a ceramic, e.g. alumina, zirconia or a blend thereof.

7. The prosthesis of any one of claims 1-6,
wherein the bone anchor (100) is formed as a screw comprising a body (101), with threads (104), wherein the body (101) comprises a neck (102) on its distal end adapted for attachment to the spherical head (200),
wherein the body (101) is e.g. a cylindrical tubular body and/or
wherein the neck (102) is e.g. a conical neck.

8. The prosthesis of any one of claims 1-7,
wherein the bone anchor (100) comprises a tubular body (101) with a hollow core (103).

9. The prosthesis of claim 8,
wherein the tubular body (101) comprises a plurality of holes (105), preferably running around the circumfence of the body.

10. The prosthesis of any one of claims 7-9,
wherein the bone anchor body (101) is provided with a recess (106) at the end of the neck (102) for attachment to the spherical head (200),
wherein the recess (106) is e.g. a screwdriver recess.

11. The prosthesis of any one of claims 1-10,
wherein the bone anchor (100) is made from metal, e.g. from titanium or a titanium alloy such as titanium-aluminium-niobium.

12. The prosthesis of claim 11,
wherein the metal is at least partially coated with a porous layer, in particular a porous layer of metal, e.g. titanium, and/or ceramic material, e.g. hydroxy-apatite.

## Patentansprüche

1. Komplettschulterprothese des reversen Typs, welche aufweist:
(i) eine konkave Komponente (300), die zum Anbringen am proximalen Humerus ausgelegt ist, und
(ii) eine konvexe Komponente, die einen kugelförmigen Kopf (200) und einen zum Anbringen an das Scapula ausgelegten Knochenanker (100) aufweist,
wobei die konkave Komponente (300) eine Ausnehmung (301) aufweist, die zur Aufnahme des kugelförmigen Kopfs (200) ausgelegt ist, und **dadurch gekennzeichnet ist, dass** die konkave Komponente (300) eine Abdeckung von etwa 180° bis etwa 200° des kugelförmigen Kopfs (200) bereitstellt.

2. Die Prothese von Anspruch 1, wobei die konkave Komponente eine Abdeckung von etwa 180° bis etwa 190° des kugelförmigen Kopfs (200) bereitstellt.

3. Die Prothese von Anspruch 1 oder 2, wobei die konkave Komponente (300) einen Körper (302) aufweist, der aus einem biokompatiblen Polymer wie etwa Polyether-Ether-Keton (PEEK) hergestellt ist.

4. Die Prothese von einem der Ansprüche 1 bis 3, wobei die konkave Komponente (300) in der Kontaktzone mit dem kugelförmigen Kopf (200) mit einem Ring (303) versehen ist, der aus einem verstärkten Material hergestellt ist, wobei das verstärkte Material zum Beispiel ein verstärktes PEEK ist.

5. Die Prothese von einem der Ansprüche 1 bis4, wobei die Rückseite der konkaven Komponente (300) zumindest teilweise mit einer porösen Schicht beschichtet ist, insbesondere einer porösen Metallschicht, zum Beispiel Titan und/oder Keramikmaterial, zum Beispiel Hydroxy-Apatit.

6. Die Prothese von einem der Ansprüche 1 bis 5, wobei der kugelförmige Kopf (200) aus Metall hergestellt ist, zum Beispiel aus einer Metalllegierung wie etwa Kobalt-Chrom oder aus einer Keramik, zum Beispiel AluminiumOxid, Zirkonium-Oxid oder einem Gemisch davon.

7. Die Prothese von einem der Ansprüche 1 bis 6, wobei der Knochenanker (100) als Schraube ausgebildet ist, die einen Körper (101) mit Gewinde (104) aufweist, wobei der Körper (101) an seinem distalen Ende einen Hals (102) aufweist, der zum Anbringen an dem kugelförmigen Kopf (200) ausgelegt ist,
wobei der Körper (101) zum Beispiel ein zylindrischer rohrförmiger Körper ist und/oder wobei der Hals (102) zum Beispiel ein konischer Hals ist.

8. Die Prothese von einem der Ansprüche 1 bis 7, wobei der Knochenanker (100) einen rohrförmigen Körper (101) mit einem hohlen Kern (103) aufweist.

9. Die Prothese von Anspruch 8, wobei der rohrförmige Körper (101) mehrere Löcher (105) aufweist, die bevorzugt um den Umfang des Körpers herum verlaufen.

10. Die Prothese von einem der Ansprüche 7 bis 9, wobei der Knochenankerkörper (101) am Ende des Halses (102) zum Anbringen an dem kugelförmigen Kopf (200) mit einer Vertiefung (106) versehen ist, wobei die Vertiefung (101) zum Beispiel eine Schraubendreher-Vertiefung ist.

11. Die Prothese von einem der Ansprüche 1 bis 10, wobei der Knochenanker (100) aus Metall hergestellt ist, zum Beispiel aus Titan oder einer TitanLegierung wie etwa Titan-Aluminium-Niob.

12. Die Prothese von Anspruch 11, wobei das Metall zumindest teilweise mit einer porösen Schicht beschichtet ist, insbesondere einer porösen Metallschicht, zum Beispiel Titan und/oder Keramikmaterial, zum Beispiel Hydroxy-Apatit.

## Revendications

1. Prothèse totale d'épaule de type inversé comprenant :
(i) un composant concave (300) adapté pour être fixé à l'humérus proximal, et
(ii) un composant convexe comprenant une tête sphérique (200) et une ancrage osseuse (100) adaptée pour être fixée à l'omoplate,
dans lequel le composant concave (300) comprend un évidement (301) adapté pour recevoir la tête sphérique (200) et **caractérisé en ce que** le composant concave (300) fournit une couverture d'environ 180° à environ 200° de la tête sphérique (200).

2. Prothèse selon la revendication 1,
dans laquelle le composant concave fournit une couverture d'environ 180° à environ 190° de la tête sphérique (200).

3. Prothèse selon la revendication 1 ou 2,
dans laquelle le composant concave (300) a un corps (302) fait d'un polymère biocompatible tel que le polyéther éther cétone (PEEK).

4. Prothèse selon l'une quelconque des revendications 1 à 3,
dans laquelle le composant concave (300) est pourvu d'un anneau (303) fait d'un matériau renforcé dans la zone de contact avec la tête sphérique 200,
dans laquelle le matériau renforcé est par exemple un PEEK renforcé.

5. Prothèse selon l'une quelconque des revendications 1 à 4,
dans laquelle la face arrière du composant concave (300) est au moins partiellement revêtue d'une couche poreuse, en particulier une couche poreuse en métal, par exemple en titane, et/ou en matériau céramique, par exemple en hydroxy-apatite.

6. Prothèse selon l'une quelconque des revendications 1 à 5,
dans laquelle la tête sphérique (200) est réalisée en métal, par exemple en alliage métallique tel que le cobalt-chrome, ou en céramique, par exemple en alumine, en zircone ou en un mélange de celles-ci.

7. Prothèse selon l'une quelconque des revendications 1 à 6,
dans laquelle l'ancrage osseuse (100) est formé comme une vis comprenant un corps (101), avec des filetages (104), dans laquelle le corps (101) comprend un col (102) sur son extrémité distale adapté pour la fixation à la tête sphérique (200),
dans laquelle le corps (101) est par exemple un corps tubulaire cylindrique et/ou
dans laquelle le col (102) est par exemple un col conique.

8. Prothèse selon l'une quelconque des revendications 1 à 7,
dans laquelle l'ancrage osseuse (100) comprend un corps tubulaire (101) avec une âme creuse (103).

9. Prothèse selon la revendication 8,
dans laquelle le corps tubulaire (101) comprend une pluralité de trous (105), s'étendant de préférence autour de la circonférence du corps.

10. Prothèse selon l'une quelconque des revendications 7 à 9,
dans laquelle le corps d'ancrage osseux (101) est pourvu d'un évidement (106) à l'extrémité du col (102) pour la fixation à la tête sphérique (200), dans laquelle l'évidement (106) est par exemple un évidement de tournevis.

11. Prothèse selon l'une quelconque des revendications 1 à 10,
dans laquelle l'ancrage osseuse (100) est réalisé en métal, par exemple en titane ou en un alliage de titane tel que le titane-aluminium-niobium.

12. Prothèse selon la revendication 11,
dans laquelle le métal est au moins partiellement revêtu d'une couche poreuse, en particulier une couche poreuse de métal, par exemple de titane, et/ou de matériau céramique, par exemple d'hydroxy-apatite.
